# EUROPEAN PATENT APPLICATION

(11) **EP 1 386 599 A1**
(43) Date of publication of application: **04.02.2004**
(21) Application number: 02715830.2
(22) Date of filing: 18.01.2002
(51) Int. Cl.: A61K 7/00, A61K 7/02, C09D 5/06, B01F 3/12

(54) **PROCESS FOR PRODUCING DRY WATER**

(30) Priority: 18.01.2001 JP 2001010739
(71) Applicant: SHISEIDO COMPANY, LTD., Chuo-ku, Tokyo 104-8010 (JP)
(72) Inventor: OKA, Takashi, SHISEIDO RES. CENTER(SHIN-YOKOHAMA), Yokohama-shi, Kanagawa, 224-8558 (JP); KOGA, Nobuyoshi, SHISEIDO RES. CEN(SHIN-YOKOHAMA), Yokohama-shi, Kanagawa, 224-8558 (JP); TAKASU, Emiko, SHISEIDO RES. CEN.(SHIN-YOKOHOMA), Yokohama-shi, Kanagawa 224-8558 (JP); YANAKI, Toshio, SHISEIDO RES. CEN.(SHIN-YOKOHOMA), Yokohama-shi, Kanagawa 224-8558 (JP)
(74) Representative: Quantin, Bruno
(86) International application number: PCT/JP2002/000358
(87) International publication number: WO 2002/056844

(57) **Abstract**

A production method of dry water containing an aqueous ingredient coated with a hydrophobic powder to form a powder state capable of liquefying upon embrocation at the time of use, wherein the dry water is produced by charging a hydrophobic powder and an aqueous ingredient into a hollow container forming a hydrophobic enclosed space in the inside thereof, followed by agitating at a high speed in the enclosed hydrophobic hollow container to form the aqueous ingredient to fine aqueous droplets, and then allowing the surfaces of the fine aqueous droplets to be uniformly adsorbed with the hydrophobic powder, whereby the dry water in the form of a powder state, but is capable of being liquefied upon embrocation at the time of use to be simply produced in a large scale.

## Description

### TECHNICAL FIELD

The present invention relates to a method for producing dry water capable of simply, producing dry water, in a large amount, comprised of an aqueous ingredient in the state of fine aqueous droplets uniformly coated on the surfaces thereof with oriented hydrophobic powder to form a powder state and capable of liquefying when embrocated at the time of use.

### BACKGROUND ART

In the past, powder substances called "dry water" have been comprised of water or an aqueous ingredient other than water coated with a hydrophobic powder or a powder hydrophobically treated etc. to form a powder which is capable of being liquefied when embrocated at the time of use. This dry water is used in the field of cosmetic composition as a powder cosmetic composition and is in the stage of being commercialized as powder water-based paints etc. in the ink and printing industry.

However, in the past, an enclosed type mixing apparatus such as a mixer has been used to crush, mix, and agitate two or more types of powder ingredients to, for example, break up aggregates of the powder ingredients. Using these enclosed type mixers to mix and agitate contents of natures incompatible with each other, that is, water and a powder, was not practiced at all in the industry. Rather, the idea that such mixing was useless from the viewpoint of labor, economy, resources, etc. had broadly pervaded the industry as common knowledge, and therefore, such mixing and agitation had not even been attempted.

Under these circumstances, since the dry water is by nature obtained by uniformly coating an aqueous ingredient with a hydrophobic powder to form a powder, uniform mixing of the aqueous ingredient and the hydrophobic powder is difficult. For example, for powder cosmetic compositions, sample mills, magnetic stirrers, dispersers, homomixers, Polytron mixers, three-one motors and other small-sized mixers have used to produce the powder cosmetic compositions in small amounts at a time. These mixers were all usually used in open systems, and therefore, there were limits to the agitation and mixing speed etc. in terms of preventing the contents from flying out from the containers and mass production was difficult.

Further, in the past, it was difficult to formulate a powder of a laminating agent, pearling agent, or the like (hydrophobic powder) into water-based cosmetic compositions such as cosmetic water, beauty lotions, uniformly with a good dispersion. If the formulation amount is increased, these ingredients cause precipitating or floating and problems in the production process occurs.

Further, even if ingredients unstable in the presence of water or ingredients having a deleterious effect on the physical properties of the product in the dissolved state in water (hereinafter referred to all together as "unstable ingredients in the presence of water") are formulated, stably formulation of these ingredients to mass produce a powder cosmetic composition capable of sufficiently exhibiting the functions of the ingredients is desired. Such unstable ingredients in the presence of water are water-soluble ingredients formulated into cosmetic compositions, pharmaceuticals, etc. as active ingredients, etc. and requiring the intervention of water in order to exhibit their functions, but if formulated into a water-based or emulsion type cosmetic composition as is in a state unstable in the presence of water, for example, due to contact with water or the effects of light, heat, etc., break down, lose activity, precipitate as crystals, discolor or fade, or produce a foul odor, separate, coagulate, thicken, or the like. As such ingredients, for example, active ingredients such as whitening agents, anti-inflammatory agents, antibacterial agents, hormones, vitamins, enzymes, antioxidants, plant extracts may be exemplified.

### DISCLOSURE OF THE INVENTION

The present invention was made in consideration of this situation and has an object to provide a method for producing a dry water capable of simply producing, in a large amount, dry water of a powder state capable of being liquefied by embrocation at the time of use.

The inventors engaged in intensive study to solve the above problems and, as a result, found that, by mixing and agitating water and a powder, that is, contents of natures incompatible with each other, which had been considered useless in terms of labor, economy, resources, etc. and had not even been attempted, under specific conditions using a enclosed type mixer (or internal mixer), surprisingly, completely different from the conventional common knowledge, it is possible to easily cause the surfaces of the aqueous ingredient in the aqueous droplet state to be uniformly coated with the hydrophobic powder and possible to mass produce dry water whereby the present invention was completed.

That is, in accordance with the present invention, there is provided a method for producing dry water composed of an aqueous ingredient coated with a hydrophobic powder to form a powder state capable of liquefying upon embrocation at the time of use, comprising charging a hydrophobic powder and an aqueous ingredient into a hollow container forming a hydrophobic enclosed space in the inside thereof, followed by agitating at a high speed in the hydrophobic hollow container to form fine droplets, then causing the surfaces of the fine aqueous droplets to be uniformly adsorbed with the hydrophobic powder.

In the above production method, the average particle size of the dry water is preferably 5 mm or less, more preferably 1 mm or less particularly preferably 10 nm to 1 mm.

In the above production method, preferably a homogenizer, rocking mixer, rocking mixer with a crushing function, shaker, V-type mixer, air agitation mixer, horizontal cylindrical type mixer, double cone type mixer, ribbon type mixer or high speed fluid mixer is used to agitate the ingredients at a high speed in the enclosed hydrophobic hollow container to convert the aqueous ingredient into fine aqueous droplets.

In accordance with the present invention, there is also provided a powder cosmetic composition containing the dry water obtained by the above production method.

In accordance with the present invention, there is further provided a powder paint containing the dry water obtained by the above production method.

### BEST MODE FOR CARRYING OUT THE INVENTION

The present invention will now be explained in detail.

In the present invention, the "dry water" broadly means a powder-like substance composed of water or an aqueous ingredient other than water coated with a hydrophobic powder or a powder hydrophobically treated, etc. to form a powder which is capable of being liquefied when embrocated at the time of use or the aggregate of such powder.

The hydrophobic powder used in the present invention includes not only those where the powder itself is hydrophoblic, but also hydrophilic powder etc. where the surface of the powder is hydrophobically treated, obtained by hydrophobically treating the powder. Here, "hydrophobic" means the property of the interaction with water being small and the affinity with water being small. The hydrophobic powder used in the present invention is a powder where the average primary particle size is preferably 50 nm or less, more preferably 10 to 30 nm, and a powder where the surface area is preferably at least 50 m²/g, more preferably 60 to 1000 m²/g.

As the hydrophobic powder used in the present invention, specifically an organic powder such as a polyamide resin powder (nylon powder), polyethylene powder, polymethyl methacrylate powder, polystyrene powder, copolymer resin powder of styrene and acrylic acid, benzoguanamine resin powder, polytetrafluoroethylene powder, cellulose powder, or silicone powder such as trimethylsilsesquioxane powder etc. may be mentioned.

As the powder ingredient of the hydrophobically treated powder usable in the present invention, for example, inorganic powders such as talc, kaolin, mica, sericite, dolomite, phlogopite, synthetic mica, lepidolite, biotite, lithia mica, vermiculite, magnesium carbonate, calcium carbonate, aluminum silicate, barium silicate, calcium silicate, magnesium silicate, strontium silicate, tungstenic acid metal salts, magnesium, silica, zeolite, barium sulfate, calcined calcium sulfate (calcined gypsum), calcium phosphate, fluorapatite, hydroxyapatite, ceramic powder, metal soap (zinc myristate, calcium palmitate, aluminum stearate, etc.), boronitride; inorganic white pigments such as titanium dioxide, zinc oxide; inorganic red pigments such as iron oxide (bengara), iron titanate; inorganic brown pigments such as γ-iron oxide; inorganic yellow pigments such as yellow iron oxide, yellow orcher; inorganic black pigments such as black iron oxide, carbon black, lower titanium oxide; inorganic violet pigments, such as mango violet, cobalt violet; inorganic green pigments such as chromium oxide, chromium hydroxide, cobalt titanate; inorganic blue pigments such as ultramarine, iron blue; pearl pigments such as titanium oxide coated mica, titanium oxide coated bismuth oxychloride, titanium oxide coated talc, colored titanium oxide coated mica, bismuth oxychloride, fish scale flakes; and metal power pigments such as aluminum powder, copper powder; etc. may be mentioned. In the present invention, these hydrophobically treated powder ingredients may be used.

Among these hydrophobic powders, as a hydrophoblic gloss ingredient usable as a laminating agent or pearling agent, panel pigments such as titanium oxide coated mica, titanium oxide coated bismuth oxychloride, titanium oxide coated talc, colored titanium oxide coated mica, bismuth oxychloride, fish scale flakes, or otherwise polyethylene terephthalate, polymethyl methacrylate multilayer film powder, etc. may be mentioned.

Among these, as a hydrophobic powder, hydrophobic silicic anhydride composed of particulate silicic anhydride hydrophobically treated on the surface thereof is preferably used. This hydrophobic silicic anhydride is particularly preferable from the viewpoint of uniformly coating an aqueous ingredient having a surface area of 60 m²/g or more.

As the method of hydrophobic treatment, any method which can impart water repellency to a powder may be used. The method is not specifically limited, but, for example, ordinary surface treatment methods such as the vapor phase method, liquid phase method, autoclave method, mechanochemical method, etc. may be used.

For example, when the treatment is carried out by adding a hydrophobic treatment agent to a raw material powder, it is possible to dilute the treatment agent in a suitable solvent (e.g., dichloromethane, chloroform, hexane, ethanol, xylene, volatile silicone, etc.) for addition or to directly add the treatment agent. To agitate and mix the powder and the treatment agent, a ball mill, faujasite ball mill, vibration ball mill, attritor, pot mill, rod mill, pan mill, homomixer, homodisperser, Henschel mixer, Nauta mixer, etc. may be used. In addition, the method for polymerizing a cyclic organosiloxane on the powder surface at a low temperature of 100°C or less by a vapor phase reaction utilizing the activity of the powder surface (see Japanese Examined Patent Publication (Kokoku) No. 1-54380), the method of adding pendant groups such as glyceryl-α-monoallylether to the Si-H portions of the silicone polymer of the surface after the above method (see Japanese Examined Patent Publication (Kokoku) No. 1-54381), etc. may be used.

The hydrophobic treatment agent is not particularly limited, but low viscosity to high viscosity oil-like polysiloxane treated powder such as a fatty acid dextrin treated powder, trimethylsiloxysilicate treated powder, fluorine-modified trimethylsiloxysilicate treated powder, methylphenylsiloxysilicate treated powder, fluorine-modified methylphenylsiloxysilicate treated powder, methyl polysiloxane, dimethyl polysiloxane, trimethyl polysiloxane, diphenyl polysiloxnae, methylphenyl polysiloxane; silyl compound or fluorine-substituent treated powder such as gum-like polysiloxane treated powder, methylhydrogen polysiloxane treated powder, fluorine-modified methylhydrogen polysiloxane-treated powder, methyl trichlorosilane, methyl trialkoxysilane, hexamethyl disilane, dimethyl dichlorosilane, dimethyl alkoxysilane, trimethyl chlorosilane, trimethyl alkoxysilane; organic modified silane or fluorine substituent treated powder such as ethyl trichlorosilane, ethyl trialkoxysilane, propyl trichlorosilane, propyl trialkoxysilane, hexyl trichlorosilane, hexyl trialkoxysilane, long-chain alkyl trichlorosilane, long-chain alkyl triethoxysilane; and amine-modified polysiloxane treated powder, fluorine-modified polysiloxane treated powder, fluoroalkyl phosphoric acid treated power, etc. may be mentioned. Further, perfluoroalkylphosphoric acid ester diethanolamine salts, perfluoroalkylsulfuric acid ester diethanolamine salts, perfluoroalkyl-carboxylic acid ester diethanolamine salts, perfluoropolyether dialkylphosphoric acid and its salts, perfluoropolyether dialkylsulfuric acid and its salts, perfluoropolyether dialkylcarboxylic acid and its salts, etc. may be used.

These hydrophobic powders may be used alone or in any combination thereof. Further, it is possible to suitably formulate a powder ingredient usable in the fields to which dry water is applied (for example, powder cosmetic compositions, powder water-based paints, etc.) based upon those fields. The invention is not limited to the above-exemplified powder ingredients.

As the aqueous ingredient, in addition to water, it is possible to use any water-soluble ingredients usable in the fields to which dry water is applied (for example, powder cosmetic compositions, powder water-based paints, etc.), based upon those fields.

As an example for the case of applying the dry water to a powder cosmetic composition, any water-soluble ingredient generally used as a cosmetic composition can be used. As the water-soluble ingredient, for example, a water-soluble polymer, water-soluble active ingredient, etc. may be mentioned, but the present invention is not limited to those illustrated. Further, an oil-in-water type emulsion composition, an aqueous ingredient in which an oil ingredient is encapsulated and held internally, etc. may be mentioned.

The water-soluble polymer is not particularly limited so long as it can be formulated into a cosmetic composition. For example, natural water-soluble polymers, semi-synthesized water-soluble polymers, synthesized water-soluble polymers, inorganic water-soluble polymers, etc. may be mentioned.

As a natural water-soluble polymer, for example, plant-based water-soluble polymers such as gum arabic, gum tragacanth, galactan, guar gum, locust bean gum, carob gum, gum karaya, carrageenan, pectin, mannan, agar, quince seed, algae colloid, starch (rice, corn, potato, wheat); microorganism-based water-soluble polymers such as xanthane gum, hyaluronic acid, dextran, succinoglucan, curdlan, pullulan, animal-based water-soluble polymers such as collagen, casein, albumin, gelatin; etc. may be mentioned.

As a semi-synthesized water-soluble polymer, for example, acetylated hyaluronic acid; starch-based water-soluble polymers such as carboxymethyl starch, methylhydroxypropyl starch; cellulose-based water-soluble polymers such as methylcellulose, nitrocellulose, ethylcellulose, methylhydroxypropyl-cellulose, hydroxyethylcellulose, sodium cellulose sulfate, hydroxypropylcellulose, sodium carboxy-methylcellulose (CMC), crystalline cellulose, cellulose powder; alginic acid-based water-soluble polymers such as sodium alginate, alginic acid propylene glycol esters; etc. may be mentioned.

As a synthesized water-soluble polymer, for example vinyl-type water-soluble polymers such as polyvinyl alcohol, polyvinyl methyl ether, polyvinyl pyrrolidone, carboxyvinyl polymer (tradename: "Hibiswako (made by Wako Pure Chemicals"); polyoxyethylene-based water-soluble polymers such as polyethylene glycol (molecular weight 20,000, 600,000, 4,000,000); acryl-based water-soluble polymers such as polyoxyethylene polyoxypropylene copolymer-based water-soluble polymers; sodium polyacrylate, polyethyl acrylate, polyacrylamide; polyethyleneimine; cationic polymers, etc. may be mentioned.

As an inorganic water-soluble polymer, for example, bentonite, AlMg silicate (tradename: Veegum"), Laponite, hectrite, etc. may be mentioned.

The water-soluble polymer may be used alone or in any combination thereof. Among these, microorganism-based water-soluble polymers such as xanthane gum, hyaluronic acid, dextran, succinoglucan, curdlan, pullulan; cellulose-based water-soluble polymers such as methylcellulose, nitrocellulose, ethylcellulose, methylhydroxypropylcellulose, hydroxyethylcellulose, sodium cellulose sulfate, hydroxypropylcellulose, sodium carboxymethylcellulose (CMC), crystalline cellulose, cellulose powder; alginic acid-based water-soluble polymers such as sodium alginate, alginic acid propylene glycol esters; etc. are preferably used.

When applying the dry water to a cosmetic composition, it is possible to formulate, in addition to the above ingredients, various types of additive ingredients used for ordinary cosmetic compositions such as a humectant, fragrance, preservative, salt, etc. in a range not obstructing the effect of the present invention. Further, a water-soluble active ingredient may also be formulated. As such as ingredient, an ingredient unstable in the presence of water may be illustrated such as a whitening agent, anti-inflammatory agent, antibacterial agent, hormone, vitamin, enzyme, antioxidant, plant extract, or other active ingredient may be mentioned. The powder cosmetic composition obtained by the method of the present invention can stably contain such an ingredient unstable in the presence of water over a long period.

As a whitening agent, hydroxyquinone derivative such as albutin, kojic acid, L-ascorbic acid and its derivatives, tranexamic acid and its derivatives, etc. may be illustrated. L-ascorbic acid is generally called vitamin C. Due to its strong reducing action, it has a cell respiratory action, enzyme activating action, and collagen forming action and has a melanin reducing action. As an L-ascorbic acid derivative, for example, L-ascorbic acid monoesters such as L-ascorbic acid monophosphoric acid ester, L-ascorbic acid-2-sulfuric acid ester; L-ascorbic acid glucosides such as L-ascorbic acid-2-glucoside; or salts of the same (L-ascorbic acid magnesium phosphate etc.) etc. may be mentioned.

As a tranexemic acid derivative, a dimer of tranexemic acid (e.g., hydrochlorate trans-4-(tran-aminomethylcyclohexanecarbonyl)aminomethylcyclohexacarboxylic acid etc.), an ester of tranexemic acid and hydroquinone (e.g., trans-4-aminomethylcyclohexanecarboxylic acid 4'-hydroxyphenylester etc.), an ester of tranexemic acid and gentisic acid (e.g., 2-(trans-4-aminomethylcyclohexyl-carbonyloxy)-5-hydroxybenzoic acid and its salts etc.), an amide of tranexemic acid (e.g., methylamide trans-4-aminomethylcyclohexanecarboxylate and its salts, trans-4-(P-methoxybenzoyl)aminomethyl-cyclohexanecarboxylic acid and its salts, trans-4-guanidinomethylcyclohexanecarboxylic acid and its salts, etc.), etc. may be mentioned.

As an anti-inflammatory agent, for example, a glycyrrhizinic acid salt (e.g., dipotassium glycyrrhizinate, ammonium glycyrrhizinate, etc.), allatoin, etc. may be mentioned.

As an antibacterial agent, for example resorcin, sulfur, salicylic acid, etc. may be mentioned.

As a hormone, for example, oxytocin, corticotropin, vasopressin, secretin, gastrin, calcitonin, etc. may be mentioned.

As a vitamin, for example, vitamin B₆ derivatives such as vitamin B₆, vitamin B₆ hydrochlorate; nicotinic acid derivatives such as nicotinic acid, nicotinamide; pantothenyl ethyl ether, etc. may be mentioned.

As an enzyme, for example, trypsin, lysozyme chloride, chemotrypsin, semialkali protease, serapeptase, lipase, hyaluronidase, etc. may be mentioned.

As an antioxidant, thiotaurin, glutathion, catechin, albumin, ferritin, metallothionein, etc. may be mentioned.

As a plant extract, tea extract, *rosa roxburghii plena* extract, *scutellaria* root extract, *houttuynia* herb powder, phellodendron bark extract, sweet clover extract, white nettle extract, glycyrrhiza extract, peony root extract, soap wort extract, sponge gourd extract, cinchona extract, saxifrage extract, sophora root extract, nuphar extract, fennel extract, *primula sieboldii* extract, rose extract, rehmannia root extract, lemon extract, lithospermum extract, aloe extract, calamus rhizome extract, eucalyptus extract, horse tail extract, sage extract, thyme extract, seaweed extract, cucumber extract, clove extract, raspberry extract, melissa extract, ginseng extract, horse chestnut extract, peach extract, peach leaf extract, mulberry bark extract, cornflower extract, witch hazel extract, glycyrrhiza extract, ginkgo extract, shinleaf extract, etc. may be mentioned. Further, placenta extract, collagen, etc. may be preferably used. The present invention may contain various types of ordinary active ingredients, in addition to the above active ingredients.

In the present invention, it is also possible to use an oil-in-water type emulsion composition. In this case, the outer phase (i.e., aqueous phase) used is preferably one of the above water-soluble polymers. Here, it is also possible to formulate humectant such as 1,3-butyleneglycol, glycerin; chelating agent such as metaphosphoric acid salt, edetic acid salt; or plant extracts such as yarrow extract, witch hazel extract. In addition, it is possible to formulate any ordinary water-soluble ingredients.

On the other hand, the inner phase (i.e., oil phase) is not particularly limited so long as it is an oil ingredient generally usable in cosmetic compositions. For example, liquid oils and fats such as avocado oil, evening primrose oil, turtle oil, macademia nut oil, corn oil, mink oil, olive oil, rapeseed oil, eggyoke oil, sesame seed oil, persic oil, wheat germ oil, sasanqua oil, castor oil, linseed oil, safflower oil, cottonseed oil, perilla oil, soybean oil, peanut oil, tea seed oil, kaya oil, rice bran oil, Chinese tung oil, Japanese tung oil, jojoba oil, germ oil, triglycerin, glyceryl trioctanoate, pentaerythrityl tetraoctanoate, glyceryl triisopalmitate; solid oils and fats such as cacao oil, coconut oil, hydrogenated coconut oil, palm oil, palm nut oil, Japan wax nut oil, hydrogenated oil, Japan wax, hydrogenated castor oil; waxes such as beeswax, candelilla wax, cotton wax, carnauba wax, bayberry wax, insect wax, spermaceti, montan wax, rice bran oil, lanolin, kapok wax, lanolin acetate, liquefied lanolin, sugarcane wax, isopropyl lanolin fatty acid, hexyl laurate, reduced lanolin, jojoba wax, hard lanolin, shellac wax, POE lanolin alcohol ether, POE lanolin alcohol acetate, POE cholesterol ether, polyethylene glycol lanolin fatty acid, POE hydrogenated lanolin alcohol ether; hydrocarbon oils such as liquid paraffin, ozokerite, squalene, pristane, paraffin, ceresin, squalane, vaseline, microcrystalline wax; synthetic ester oils such as isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, 12-cholesterol hydroxystearate, ethylene glycol di-2-ethylhexanoate, dipentaerythrite fatty acid ester, N-alkylene glycol monoisostearate, neopentyl glycol dicaprate, diisostearyl malate, glyceryl di-2-heptyl undecanoate, trimethylolpropane tri-2-ethylhexanoate, trimethylolpropane triisostearate, pentaerythritol tetra-2-ethylhexanoate, glyceryl tri-2-ethylhexanoate, trimethylolpropane triisostearate, cetyl-2-ethylhexanoate, 2-ethylhexyl palmitate, glyceryl trimyristate, glyceryl tri-2-heptyl undecanoate, castor oil fatty acid methyl ester, oleic acid oil, cetostearyl alcohol, acetoglyceride, 2-heptylundecyl palmitate, 2-octyldodecyl-N-lauroyl-L-glutamate, ethyl laurate, 2-hexyldecyl myristate, 2-hexyldecyl palmitate, ethyl acetate, butyl acetate, amyl acetate, triethyl citrate, chain type polysiloxanes such as dimethyl polysiloxane, methylphenyl polysiloxane, methylhydrogenpolysiloxane; ring type silicones such as octamethylcyclotetrasiloxane, decamethylcyclopentasiloxane, dodecamethylcyclohexasiloxane; etc. may be mentioned, but the invention is not limited to these examples. The oil ingredient may be used alone or in any combination thereof.

For the oil phase, it is also possible to make joint use of an oil gelling agent such as an organic modified clay mineral, starch fatty acid ester, dimethyl polysiloxane polymer, etc. Further, as the oil ingredient, it is also possible to use a high melting point wax such as microcrystalline wax converted to fine powder, porous powder such as magnesium carbonate high agglutinative polymer such as acrylate copolymer, etc. made to carry and absorb an oil ingredient and converted to a powder, an oil ingredient encapsulated with polymethyl methacrylate, hydrogenated gelatin, agar, etc.

In particular, by using an encapsulated oil ingredient, there are the effects that (i) it is possible to formulate a plurality of types of oil ingredients to adjust and improve the usability of the cosmetic composition and (ii) include an oil-soluble active ingredient, fragrance, etc. in a capsule to stabilize it until embrocation and use, whereby the active ingredient and fragrance ingredient are sustained.

As the encapsulating ingredient, specifically gelatin, agar, sodium alginate, agarose, rosin, gum arabic, epoxy, polyacrylamide, shellac, carboxymethylcellulose, polyvinyl alcohol, polyurethane, polystyrene, polyester, polyamide, urea, etc. are illustrated as being preferable.

The oil ingredient contained in the capsule is not particularly limited so long as it is an oil ingredient which can be formulated a cosmetic composition. For example, oil-soluble active ingredients such as retinol, tocopherol acetate, 1-menthol, hinokithiol, ethnyl estradiol, olive oil, an ultraviolet absorbent, or fragrance etc. may be mentioned, but the present invention is not limited to these examples. These oil ingredients may be used alone or in any combination thereof. The capsule of the oil ingredients may be produced using a known method such as the coacervation method (Japanese Unexamined Patent Publication (Kokai) No. 1-266846, etc.)

An oil-in-water type emulsion composition can be produced by an ordinary method. The method of emulsification is not particularly limited. For example, the method of warming the oil phase and the aqueous phase to about 70°C respectively and gradually adding the warmed aqueous phase to the oil phase, emulsifying the resultant mixture with an emulsifier, then allowing the mixture to cool to room temperature or another method may be mentioned, but the invention is not limited to this method.

The aqueous ingredient may be used alone or in any combination thereof.

The production method of the present invention is characterized by the points of charging the hydrophobic powder and the aqueous ingredient into a hollow container forming a hydrophobic enclosed space, then agitating the mixture at a high speed in the enclosed hydrophobic hollow container to convert the aqueous ingredient to the fine aqueous droplets and make the surfaces of the fine aqueous droplets uniformly adsorb the hydrophobic powder to form a fine powder substance.

Here, the "hydrophobic enclosed space" means not the hollow container itself for agitating and mixing the materials being hydrophobic (for example, resins such as Teflon, polypropylene, polyethylene terephthalate), but also the inside walls of the container contacting the agitated and mixed materials being made hydrophobic surfaces or the container being closed to an extent, whereby the contents do not fly outside at the time of high speed agitation and mixture. By making a hydrophobic space in this way, when agitating and mixing the hydrophobic powder and the aqueous ingredient, it is possible to prevent the separation of the water due to the higher wettability of the inside walls of the container by the aqueous ingredient, raise the agitating and mixing force, and sufficiently form a powder.

In the hollow container forming the hydrophobic enclosed space, the contents in the inside space are agitated at a high speed under conditions to convert the contents, that is, the aqueous ingredient, to fine aqueous droplets and make their surfaces uniformly adsorb the hydrophobic powder. This high speed agitation is, for example, an agitation at a linear verocity (or peripheral speed) of 100 m/sec or less, more preferably 10 to 50 m/sec. It is believed that the aqueous ingredient is converted to fine aqueous droplets since the high speed agitation induces turbulence in the inside space, the aqueous ingredient strikes the inside walls of the container and bounces back, or this is repeated to thereby form fine aqueous droplets or the droplets strike each other to form fine aqueous droplets. In the present invention, the fine aqueous droplets are preferably formed so that the average size (particle size) becomes about 5 mm or less in the state where the later explained hydrophobic powder is adsorbed. Since the adsorption of the hydrophobic powder is a coating in an extremely thin state, in the present invention the particle size of the fine aqueous droplets and the particle size of the fine powder-like substance may be deemed to be substantially the same. This particle size becomes the average particle size of the dry water. If the particle size is more than 5 mm, the uniform orientation of the hydrophobic powder to the surfaces of the fine droplets becomes difficult and obtaining dry water becomes difficult. Note that the "average particle size becomes 5 mm or less" includes the case where fine aqueous droplets combine at the time of high speed agitation and mixture to form single drops and the particle size of the fine powder-like substance in the state where the combined droplets as a whole adsorb the hydrophobic powder becomes 5 mm or less. In the present invention, when these combined aqueous droplets are formed, the combined aqueous droplets are treated as single fine aqueous droplets and their surfaces are uniformly coated with the hydrophobic powder.

During the high speed agitation, substantially simultaneously with the formation of the fine aqueous droplets, the surfaces of the finely formed innumerable droplets are uniformly coated by the hydrophobic powder. There is no need for the surfaces of the aqueous droplets to completely contact the hydrophobic powder. So long as it is possible to hold the shape of the aqueous droplets, the surfaces may partially have pores. By such high speed agitation force, mass production of dry water composed of fine aqueous droplets uniformly coated on their surfaces with hydrophobic powder becomes possible. Note that if the agitation force is too large or the agitation time is too long, the powder form is no longer obtained, and therefore, it is preferable to agitate and mix by an agitation force of a range exhibiting the above action.

As the means for high speed agitation so as to convert the aqueous ingredient to the fine aqueous droplets in a enclosed hydrophobic hollow container, for example, agitation blade members for directly compulsorily agitating the inside space at a high speed or a means for agitating at a high speed the contents in the space inside the container by shaking, rotating, vibrating, etc. the container itself by outside force may be mentioned. Specifically, for example, a homogenizer (agitating by a shaft with a knife rotating at a high speed), a rocking mixer (rotary rocking type mixer with no crushing blades), a rocking mixer with a crushing function (rotary rocking type mixer with crushing blades), a shaker, a V type-mixer (agitating by rotation of a V-shaped container), an air agitation mixer, a horizontal cylinder type mixer, a double cone type mixer, a ribbon type mixer, a high speed fluid type mixer, etc. may be mentioned. Among these, a rocking mixer with a crushing function and a shaker are preferably used. When using these mixers etc., it is believed that, due to the accessory members such as crushing blades, scrapers, turbulence is caused in the container, the contents strike the inside walls of the containers, the above members, etc. and bounce back, and the aqueous ingredient is converted to fine aqueous droplets at this time. Alternatively, it is believed that the droplets collide with each other to become finer.

Therefore, agitation just by rotating a hollow cylindrical drum not having crushing blades, scrapers, or other accessory members in one direction (forward direction or reverse direction) in an inside space is not included in the method of the present invention since the aqueous ingredient and hydrophobic powder rotate along the inside walls of the hollow cylinder without causing turbulence, without the aqueous ingredient striking the inside walls of the cylinder etc. and bouncing back, and without the formation of fine aqueous droplets.

Note that when, in addition to making a hollow cylindrical drum not having accessory members rotate in one direction, jointly using the operation of rocking the drum itself, the rocking causes the contents, that is, the aqueous ingredient, to strike the top and bottom walls of the drum etc. and bounce back, whereby it can be converted to drops, and therefore this case is included in the method of the present invention.

Further, even when there are no accessory members such as crushing blades, scrapers, in the space inside the container, in the case of using a container where the inside walls of the container form a discontinuous surface at least at one location such as in a V type-mixer or a container where the inside wall surfaces have relief portions in shape, even when the container is rotated in one direction for agitation, the inside air or contents will strike the discontinuous surface or the aqueous ingredient will strike the inside walls and bounce back and fine aqueous droplets will be obtained. Therefore, such a case is included in the method of the present invention.

Further, even when using a hollow cylindrical drum having accessory members such as crushing blades, scrapers, and driving the crushing blades, scrapers, accessories without rotating the drum, driving these members causes turbulence in the drum, makes the contents strike the inside walls of the drum, and enables the aqueous ingredient to be formed into fine aqueous droplets. Therefore, such a case is also included in the method of the present invention.

The rocking mixer with a crushing function used is preferably one having a hollow cylindrical rotating drum forming a hydrophobic enclosed space and crushing blade members arranged at least at one end of the rotating drum facing the inside of the hydrophobic enclosed space. Further, when a rotating drum having, for example, a 10 to 3000 liter capacity is used, it is preferable to make the rotating drum rotate at a peripheral speed of about 0.1 to 10 m/s and make the crushing blade members rotate in a rotational direction opposite to the rotating drum at a peripheral speed of about 5 to 100 m/s. Further, the rotating drum is preferably rocked at a rocking speed of 2 to 100 spm. By using the rocking mixer with a crushing function in this way, turbulence is efficiently caused inside the hollow cylinder, the aqueous ingredient is converted to fine droplets, and the surfaces of the fine aqueous droplets adsorb the hydrophobic powder. Note that, if the inside walls of the rotating drum is provided with a plurality of scrapers in the axial direction so as to scrape the agitated materials at the time of rotation of the drum, the turbulence in the drum can be more effectively generated. In this a case, both the crushing blade members and scrapers are made hydrophobic members or are hydrophobically treated on their surfaces. As such a rocking mixer with a crushing function, for example a dry type powder mixer (made by Aichi Electric) commercially available under the RMD series etc. is preferably used, but the present invention is not limited to this.

The shaker used is preferably one which enables shaking of a hollow cylindrical container forming a hydrophobic enclosed space at least in a vertical or lateral direction. The shaker usually is designed so that the enclosed container (hollow cylindrical container) is attached to the outside and the shaking motion of the shaker is transmitted to the enclosed container directly. The hydrophobic powder and the aqueous ingredient are charged in the hydrophobic enclosed container. In the case of an enclosed container having a volume of about 10 to 500 ml, the shaker is made to shake at a shaking speed of about 100 to 600 rpm in the vertical direction or lateral direction. By shaking in this way, the materials strike the top and bottom inside walls or the left and right inside walls along with the inside air to create turbulence, the aqueous ingredient is converted to fine aqueous droplets, preferably fine aqueous droplets having an average particle size of 5 mm or less, and the surfaces of the droplets are made to adsorb the hydrophobic powder. In this case as well, the enclosed container used is one made of a hydrophobic resin (e.g., Teflon, polypropylene, polyethylene, polyethylene terephthalate, etc.) or one having at least the inside wall surfaces forming hydrophobic surfaces. As such a shaker, for example, universal shakers commercially sold under the AW series, AS series, etc. (made by Iuchi Seieido) etc. are preferably used, but the present invention is not limited to these.

In both the rocking mixer with a crushing function and the shaker, when the dry water is produced, as mentioned above, if the agitation force is too strong, a powder form will not be obtained, and therefore it is possible to obtain a uniformly coated excellent powder preparation by agitation by a suitable extent for forming aqueous droplets.

Note that, in the method of production of dry water at the time of production, space for high speed agitation is necessary and the hydrophobic powder is generally high in bulkiness, and therefore, it is preferably used in an amount of 5 to 50% in terms of raw material weight (kg) with respect to the capacity (liters) of the production container. For example, for a container having a capacity of 10 liters, it is preferable to use 0.5 to 5 kg of the raw material weight.

In the past, the agitation mixer such as the rocking mixer with a crushing function usually had a rotating drum composed of a metal such as SUS. This was used to crush and mix agglomerated powder or hydroscopic powder without forming a hydrophobic space or to mix different powders, but this was not used to produce the dry water by mixing and agitating contents of incompatible natures such as hydrophobic powder and an aqueous ingredient to uniformly coat the surfaces of the fine aqueous droplets with the hydrophobic powder.

Note that, in the production method of the present invention, the ratio of agitation and mixing of hydrophobic powder and aqueous ingredient is preferably the hydrophobic powder (hydrophobic silicic anhydride etc.):aqueous ingredient = about 0.1:99.9 to 20:80, more preferably 0.1:99.9 to 7:93, while this cannot be said generally depending on the hydrophobic powder, aqueous ingredient, production scale, etc. used. If the hydrophobic powder is too small, it is not possible to cover the surface of the aqueous ingredient to convert it to a powder and the intended powder form may no longer be able to be obtained. On the other hand, if the hydrophobic powder is too great, it becomes possible to coat the surface of a large amount of aqueous ingredient to convert it to a powder form, but liquefaction becomes difficult even with embrocation at the time of use and the feeling becomes unpreferable. Note that, when formulating a laminating agent or pearling agent, it is preferably formulated in an amount of 2 to 50 parts by weight, more preferably 5 to 20 parts by weight, based upon 100 parts by weight of the total weight of the hydrophobic powder.

When the dry water is applied to a powder water-based paint, for example, by using a color as the aqueous ingredient and coating this uniformly with a hydrophobic powder, there is the advantage that the powder form paint can be conveniently applied by a brush etc. to a wall, paper, etc. just by embrocation.

According to the present invention, for the first time, a technical technique never even conceived of in the industry, that is, agitating and mixing a hydrophobic powder and aqueous ingredient at a high speed in a hydrophobic enclosed space to convert the aqueous ingredient to fine aqueous droplets and uniformly coat the surfaces of the fine aqueous droplets by the hydrophobic powder, is employed and it becomes possible to produce the dry water in much larger volumes and more simply compared with the past.

Further, there are provided a powder cosmetic composition superior in long-term storage stability of a product and able to stably hold an ingredient, which is unstable in the presence of water, which had been considered difficult to formulated into a conventional cosmetic composition, in a composition stably over a long period of time and a powder paint capable of being conveniently and easily used by just embrocation by a brush etc. on a paper surface or wall surface as it is.

### EXAMPLES

The present invention will now be explained in further detail based on the Examples, but the present invention is not limited to the following Examples Note that the amounts formulated all show % by weight.

Prior to the Examples, the test methods and evaluation methods used in the present invention will be explained.

### Evaluation of Preparation Process

When producing samples by the formulations shown in the following tables, whether the samples have been prepared in the powder form is evaluated from the appearance by the following criteria:

### Evaluation

○: Prepared in the powder form
Δ: Souffle-like preparation
×: Separated into two layers of powder phase and aqueous phase with preparation therefore incomplete

### Active Ingredient (Ingredient Unstable in Presence of Water) Stability Test

Samples of the Examples of the formulations shown in the following tables were measured for residual amounts of the active ingredients when stored at 40°C (one month, three months, and six months) by high pressure liquid chromatography (HPLC) and the residual rates were examined from the results.

### Long Term Storage Stability Test of Product

Samples of the examples of the formulations shown in the following tables were stored for six months at 0°C, room temperature, 40°C, and exposed conditions (irradiation with sunlight) and evaluated by the following criteria:

### Evaluation

ⓞ: Cosmetic did not change
○: Some adherence of powder or drops of water on container
Δ: Some separation of water
×: Remarkable separation of water and destruction of preparation

### Example 1

A powder cosmetic composition was produced by the composition shown in the following Table 1. The preparation process was evaluated by the above test methods. The results are shown in Table 1. Note that in Table 1, the dimethylsilicon oil treated silicic anhydride used was "Aerosil R-812S" (made by Nippon Aerosil, surface area 220 m²/g).

**Table 1**

| Formulation ingredients | | Ex. 1 |
|---|---|---|
| (1) Dimethylsilicon oil treated silicic anhydride | | 5.00 |
| (2) Edetic acid salt | | 0.20 |
| (3) 1,3-butyleneglycol | | 20.00 |
| (4) Preservative | | q.s. |
| (5) Tranexamic acid | | 1.00 |
| (6) Purified water | | Bal. |
| Preparation process | High speed agitation time | |
| | 10 min | × |
| | 20 min | × |
| | 30 min | ○ |
| | 60 min | ○ |
| | 90 min | ○ |

### Preparation Method

The ingredients (2) to (6) were mixed to prepare cosmetic water (aqueous ingredient) by an ordinary method, then the cosmetic water (aqueous ingredient) and ingredient (1) were placed into a hydrophobic container (volume 0.2 liter) which was then shaken by hand to mix them. Next, the container was set in a shaker (made by Iuchi Seieido) which was run at a shaking speed of 350 rpm to stir the ingredients at a high speed in the vertical direction. The ability to prepare the powder cosmetic composition over time was evaluated visually based on the above evaluation method.

As is clear from the results shown in Table 1, it was learned that the powder cosmetic composition of Example 1 could be prepared by 30 minutes of high speed agitation. Note that the powder cosmetic composition thus obtained was an agglomerate of innumerable fine powder substances having a size of about 0.3 mm (dry water).

### Example 2

A powder cosmetic composition was produced by the composition shown in the following Table 2. The preparation process was evaluated by the above test methods. The results are shown in Table 2. Note that, in Table 2, the dimethylsilicon oil treated silicic anhydride^{(*)} used was "Aerosil RY200S" (made by Nippon Aerosil, surface area 80 m²/g), while the silicon-based compound^{(**)} used was "Silicon Powder E506-W" (made by Toray Dow Corning Silicone).

**Table 2**

| Formulation ingredients | | Ex. 2 |
|---|---|---|
| (1) Dimethylsilicon oil treated silicic anhydride^{(*)} | | 4.00 |
| (2) Silicon-based compound^{(**)} | | 5.00 |
| (3) Edetic acid salt | | 0.10 |
| (4) Citric acid | | 0.08 |
| (5) Sodium citrate | | 0.12 |
| (6) 1,3-butyleneglycol | | 10.00 |
| (7) Glycerin | | 10.00 |
| (8) Preservative | | q.s. |
| (9) Xanthan gum | | 0.05 |
| (10) L-ascorbic acid-2-glucoside | | 2.00 |
| (11) Potassium hydroxide | | 0.40 |
| (12) Purified water | | Bal. |
| Preparation process | High speed agitation time | |
| | 3 min | × |
| | 5 min | ○ |
| | 10 min | ○ |
| | 15 min | ○ |
| | 20 min | Δ |

### Preparation Method

The ingredients (2) to (11) were mixed to prepare cosmetic water (aqueous ingredient) by an ordinary method, then the cosmetic water (aqueous ingredient) and ingredient (1) were placed into a drum (volume of 10 liters) of a rocking mixer with a crushing function (made by Aichi Electric), then the drum of the rocking mixer with a crushing function was rotated at a peripheral speed of 0.5 m/s. This was rocked and the crushing blades were driven to stir the contents inside the drum at a high speed. The ability to prepare the powder cosmetic composition over time was evaluated visually based on the above evaluation method.

As clear from the results of Table 2, it was learned that the powder cosmetic composition of Example 2 could be prepared by 5 to 15 minutes of high speed agitation. Note that the powder cosmetic composition the obtained was an agglomerate of innumerable fine powder substances of sizes of about 0.2 mm (dry water).

### Example 3

A powder cosmetic composition was produced by the composition shown in the following Table 3. The preparation process was evaluated by the above test methods. The results are shown in Table 3. Note that, in Table 3, the dimethylsilicon oil treated silicic anhydride^{(*)} used was "Aerosil R202" (made by Nippon Aerosil, surface area 100 m²/g).

**Table 3**

| Formulation ingredients | | Ex. 3 |
|---|---|---|
| (1) Dimethylsilicon oil treated silicic anhydride^{(*)} | | 6.00 |
| (2) Edetic acid salt | | 0.05 |
| (3) Citric acid | | 0.02 |
| (4) Sodium citrate | | 0.12 |
| (5) 1,3-butyleneglycol | | 15.00 |
| (6) Polyethylene glycol 400 | | 5.00 |
| (7) Preservative | | q.s. |
| (8) Albutin | | 5.00 |
| (9) Purified water | | Bal. |
| Preparation process | High speed agitation time | |
| | 5 min | × |
| | 20 min | ○ |
| | 30 min | ○ |
| | 60 min | ○ |
| | 90 min | Δ |

### Preparation Method

The ingredients (2) to (9) were mixed to prepare cosmetic water (aqueous ingredient) by an ordinary method, then the cosmetic water (aqueous ingredient) and ingredient (1) were placed into a drum (volume of 10 liters) of a rocking mixer (without a crushing function, made by Aichi Electric), then the drum of the rocking mixer was rotated at a peripheral speed of 0.6 m/s. This was rocked to stir the contents inside the drum at a high speed. The ability to prepare the powder cosmetic composition over time was evaluated visually based on the above evaluation method.

As is clear from the results of Table 3, it was learned that the powder cosmetic composition of Example 3 could be prepared by 20 to 60 minutes of high speed agitation. Note that the powder cosmetic composition thus obtained was an agglomerate of innumerable fine powder substances having a size of about 0.2 mm (dry water).

### Example 4

A powder cosmetic composition was produced by the composition shown in the following Table 4. The preparation process was evaluated by the above test methods. The results are shown in Table 4. Note that, in Table 4, the dimethylsilicon oil treated silicic anhydride^{(*)} used was "Aerosil RY200S" (made by Nippon Aerosil, surface area 80 m²/g).

**Table 4**

| Formulation ingredients | | Ex. 4 |
|---|---|---|
| (1) Dimethylsilicon oil treated silicic anhydride^{(*)} | | 10.00 |
| (2) Edetic acid salt | | 0.10 |
| (3) Citric acid | | 0.05 |
| (4) Sodium citrate | | 0.15 |
| (5) 1,3-butyleneglycol | | 10.00 |
| (6) Glycerin | | 3.00 |
| (7) Preservative | | q.s. |
| (8) Xanthan gum | | 0.01 |
| (9) Pantothenyl ethyl ether | | 0.05 |
| (10) Potassium hydroxide | | 0.38 |
| (11) Purified water | | Bal. |
| Preparation process | High speed agitation time | |
| | 10 min | × |
| | 25 min | ○ |
| | 40 min | ○ |
| | 60 min | ○ |
| | 90 min | Δ |

### Preparation Method

The ingredients (2) to (11) were mixed to prepare cosmetic water (aqueous ingredient) by an ordinary method, then the cosmetic water (aqueous ingredient) and ingredient (1) were placed into a V-drum (volume of 3 liters) of a V-blender (made by Irie Seisakusho) and mixed, then the V-drum was rotated at a rotational speed of 30 rpm. The ability to prepare the powder cosmetic composition over time was evaluated visually based on the above evaluation method.

As is clear from the results of Table 4, it was learned that the powder cosmetic composition of Example 4 could be prepared by 25 to 60 minutes of high speed agitation. Note that the powder cosmetic composition thus obtained was an agglomerate of innumerable fine powder substances having a size of about 0.3 mm (dry water).

### Example 5

A powder cosmetic composition containing a laminating agent was produced as a hydrophobic powder by the composition shown in the following Table 5. The preparation process was evaluated by the above test methods. The results are shown in Table 5. Note that, in Table 5, the dimethylsilicon oil treated silicic anhydride used was "Aerosil R202" (made by Nippon Aerosil, surface area 100 m²/g), while the "silicic anhydride" used as "Sildex L-51" (made by Asahi Glass).

**Table 5**

| Formulation ingredients | | Ex. 5 |
|---|---|---|
| (1) Dimethylsilicon oil treated silicic anhydride | | 5.00 |
| (2) Anhydrous silicic salt | | 7.00 |
| (3) Mica | | q.s. |
| (4) Dipropylene glycol | | 10.00 |
| (5) Ion exchanged water | | Bal. |
| (6) Glycerin | | 5.00 |
| (7) Edetic acid salt | | 0.01 |
| (8) Citric acid | | q.s. |
| (9) Sodium citrate | | q.s. |
| Preparation process | High speed agitation time | |
| | 10 min | × |
| | 25 min | ○ |
| | 40 min | ○ |
| | 60 min | ○ |
| | 90 min | Δ |

### Preparation Method

The ingredients (4) to (9) were mixed to prepare cosmetic water (aqueous ingredient) by an ordinary method, then the cosmetic water (aqueous ingredient) and ingredients (1) to (3) were placed into a capsule (volume of 60 liters) of a rocking mixer with a crushing function (made by Aichi Electric), then the capsule was rotated at a peripheral speed of 0.6 m/s. This was rocked and the crushing blades were driven to agitate the contents inside the capsule at a high speed. The ability to prepare the powder cosmetic composition over time was evaluated visually based on the above evaluation method.

As is clear from the results of Table 5, it was learned that the powder cosmetic composition of Example 5 could be prepared by 25 to 60 minutes of high speed agitation. Note that the powder cosmetic composition thus obtained was an agglomerate of innumerable fine powder substances having a size of about 0.2 mm (dry water).

The powder cosmetic composition can be formulated in, for example, a body powder to obtain a body powder superior in freshness and moistness. Further, by formulating it into an aqueous phase, no phenomenon of precipitation, separation, etc. of the laminating agent is seen and a water-based cosmetic composition having an aqueous phase superior in uniform dispersion is obtained.

### Long-Term Storage Stability of Product

The powder cosmetic composition of the present invention prepared in Example 5 was evaluated for long-term storage stability of the product by the above-mentioned long-term storage stability test method. The results are shown in Table 6.

**Table 6**

| | Ex. 5 |
|---|---|
| Storage at 0°C | ○ |
| Storage at room temperature | ⓞ |
| Storage at 40°C | ○ |
| Exposed conditions | ○ |

As is clear from the results of Table 6, the powder cosmetic composition of Example 5 did not change much at all in form of preparation even after the elapse of six months under the different storage conditions. There was no problem in the product storage stability over time.

### Stability of Active Ingredient (Ingredients Unstable in Presence of Water)

The powder cosmetic composition of the present invention prepared in Examples 1 and 2 were evaluated for stability of the ingredients unstable in the presence of water by the above mentioned preparation (ingredients unstable in presence of water) stability test method. The results are shown in Table 7.

**Table 7**

| | Residual rate of ingredients unstable in presence of water in preparation (wt%) | | |
|---|---|---|---|
| | 1 month | 3 months | 6 months |
| Ex. 1 | 100 | 98 | 98 |
| Ex. 2 | 100 | 99 | 99 |
| *: Example 1 = tranexamic acid Example 2 = L-ascorbic acid-2-glucoside | | | |

As is clear from the results of Table 7, in both of the powder cosmetic compositions of Examples 1 and 2, the unstable active ingredient remained almost entirely intact even after the elapse of six months at 40°C. There was no problem in the stability of the active ingredient over time.

### Long-Term Storage Stability of Product

The powder cosmetic compositions of the present invention prepared in Examples 1 and 2 were evaluated as to the long-term storage stability of the products by the above long-term storage stability test method. The results are shown in Table 8.

**Table 8**

| | Ex. 1 | Ex. 2 |
|---|---|---|
| Storage at 0°C | ○ | ○ |
| Storage at room temperature | ⓞ | ⓞ |
| Storage at 40°C | ○ | ○ |
| Exposed conditions | ○ | ○ |

As is clear from the results of Table 8, in each of the powder cosmetic compositions of Examples 1 and 2, there was almost no change in the form of the preparation even after the elapse of six months under the different storage conditions. There was no problem in the product storage stability over time.

### Example 6

A powder water-based paint of the composition shown in the following Table 9 was produced. The preparation process of Example 6 was evaluated by the above test methods. The results are shown in Table 9. Note that in Table 9, the dimethylsilicon oil treated silicic anhydride^{(*)} used was "Aerosil R-972" (made by Nippon Aerosil, surface area 110 m²/g).

**Table 9**

| Formulation ingredients | | Ex. 6 |
|---|---|---|
| (1) Dimethylsilicon oil treated silicic anhydride^{(*)} | | 10.00 |
| (2) Color | | q.s. |
| (3) Water | | Bal. |
| Preparation process | High speed agitation time | |
| | 10 min | × |
| | 25 min | × |
| | 40 min | ○ |
| | 60 min | ○ |
| | 90 min | ○ |

### Preparation Method

The ingredients (2) and (3) were mixed to prepare a water-based paint, then the aqueous solution and ingredient (1) were placed into a hydrophobic container (volume 0.2 liter) which was then shaken by hand to mix them. Next, the container was set in a shaker (made by Iuchi Seieido) which was run at a shaking speed of 300 rpm to stir the ingredients at a high speed in the vertical direction. The ability to prepare the powder water-based paint over time was evaluated visually based on the above evaluation method.

As is clear from the results of Table 9, it was learned that the powder water-based paint of Example 6 could be prepared by 40 minutes of high speed agitation. Note that the obtained powder water-based paint was an agglomerate of innumerable fine powder substances having a size of about 0.2 mm (dry water).

### INDUSTRIAL APPLICABILITY

As explained in detail above, according to the present invention, it has become possible to produce dry water, which had been considered difficult to mass produce in the past, in mass and simply. According to the present invention, it has become possible to mass produce a powder cosmetic composition capable of stably holding unstable ingredients in a composition over a long period in the presence of water - for which formulating into a cosmetic composition had been considered difficult in the past. Further, by applying the present invention to a powder paint, mass production can also be expected.

## Claims

1. A method for producing dry water composed of an aqueous ingredient coated with a hydrophobic powder to form a powder state capable of liquefying upon embrocation at the time of use, comprising charging a hydrophobic powder and an aqueous ingredient into a hollow container forming a hydrophobic enclosed space in the inside thereof, followed by agitating at a high speed in the hydrophobic hollow container to form fine aqueous droplets, and then allowing the surfaces of the fine aqueous droplets to be uniformly adsorbed with the hydrophobic powder.

2. A production method as claimed in claim 1, wherein an average particle size of the dry water is 5 mm or less.

3. A production method as claimed in claim 2, wherein a homogenizer, rocking mixer, rocking mixer with a crushing function, shaker, V-type mixer, air agitation type mixer, horizontal cylindrical type mixer, double cone type mixer, ribbon type mixer or high speed fluid mixer is used to agitate the ingredients at a speed sufficiently high to form fine droplets of the aqueous ingredient in the horizontal cylinder and allow the surfaces to be uniformly adsorbed with a hydrophobic powder, whereby the dry water having an average particle size of 5 mm or less is produced.

4. A powder cosmetic composition comprising dry water obtained by the production method according to any one of claims 1 to 3.

5. A powder paint comprising the dry water obtained by the production method according to any one of claims 1 to 3.
